# EUROPÄISCHE PATENTSCHRIFT

(11) **EP 4 558 104 B1**
(45) Veröffentlichungstag und Bekanntmachung des Hinweises auf die Patenterteilung: **03.06.2026**
(21) Anmeldenummer: 23741701.9
(22) Anmeldetag: 13.07.2023
(51) Int. Cl.: A61F 5/02, A41C 1/10, A61F 5/03

(54) **BANDAGE ODER ORTHESE MIT BAUCHSTÜTZFUNKTION**
BANDAGE OR ORTHOSIS WITH ABDOMINAL SUPPORT FUNCTION
BANDAGE OU ORTHÈSE À FONCTION DE SUPPORT ABDOMINAL

(30) Priorität: 18.07.2022 DE 102022117881
(43) Veröffentlichungstag der Anmeldung: 28.05.2025
(73) Patentinhaber: Bauerfeind AG, 07937 Zeulenroda-Triebes (DE)
(72) Erfinder: GRUNDMANN, Madlin, 07937 Zeulenroda-Triebes (DE); BAUERFEIND-JOHNSON, Beatrix, 07937 Zeulenroda-Triebes (DE)
(74) Vertreter: Gleiss Große Schrell und Partner mbB
(86) Internationale Anmeldenummer: PCT/EP2023/069482
(87) Internationale Veröffentlichungsnummer: WO 2024/017756

(56) Entgegenhaltungen:
- EP-A1- 2 965 726
- EP-A1- 3 243 490
- US-A- 344 065
- US-A1- 2013 072 087
- US-A1- 2021 259 873
- US-A1- 2022 175 052

## Beschreibung

Die vorliegende Erfindung betrifft eine Bandage oder Orthese, die insbesondere in der Schwangerschaft eingesetzt werden kann.

Aufgrund der Gewichtszunahme, beispielsweise während der Schwangerschaft, bedingt durch das Wachstum des Kindes, kann es zu Rückenschmerzen kommen. Es kann zudem zu einer Hohlkreuzbildung kommen, da während der Schwangerschaft das Becken "verkippt" wird, um im Gleichgewicht zu bleiben. Genauer gesagt kann es zu Problemen mit den Facettengelenken im Lumbalbereich und im Becken mit dem Iliosakralgelenk (ISG) oder auch Kreuz-Darmbein-Gelenk und der Symphyse kommen. Durch das zusätzlich zu tragende Gewicht wird die Belastung auf die Wirbelsäule und das Iliosakralgelenk erhöht. Aufgrund der hormonellen Umstellung während der Schwangerschaft werden die Sehnen und Bänder in der Hüfte weicher und das Becken beginnt "auseinanderzugleiten" was mit Symphysenschmerz einhergeht.

Herkömmliche Rückenorthesen arbeiten nach einem "3-Punkt-System", dass an den anatomischen Stellen: (1) Bauchpartie (2) obere Rückenpartie und (3) untere Rückenpartie Kraft einleitet, wobei der Schwerpunkt der Krafteinleitung die Punkte 2 und 3 - also der Rücken - ist.

Die US 2022/175052 A1 offenbart eine Orthese oder Bandage für den Lumbalbereich, mit einem Grundkörper, dessen Endbereiche miteinander reversibel verbindbar sind, und mit Bauchhebe- und Zugelementen.

Das der vorliegenden Erfindung zugrunde liegende technische Problem ist die Bereitstellung einer verbesserten Bandage oder Orthese, beispielsweise Schwangerschaftsorthese, die insbesondere die Wirbelsäule und das Iliosakralgelenk sowie die Symphyse entlasten kann, das entstandene übermäßige Hohlkreuz etwas ausgleichen kann und zusätzlich das Becken stabilisiert, um die ISG-Gelenk- und Symphysenschmerzen zu lindern.

Die vorliegende Erfindung löst das ihr zugrunde liegende Problem durch den Gegenstand nach Anspruch 1.

Die vorliegende Erfindung betrifft insbesondere eine Bandage oder Orthese für den Lumbalbereich, aufweisend
- einen langgestreckten, flachen Grundkörper, aufweisend einen ersten Endbereich, einen zweiten Endbereich, einen Mittelbereich und einen ersten Zwischenbereich zwischen dem ersten Endbereich und dem Mittelbereich und einem zweiten Zwischenbereich zwischen dem zweiten Endbereich und dem Mittelbereich, wobei der erste Endbereich und der zweite Endbereich miteinander reversibel verbindbar sind,
- ein erstes langgestrecktes Bauchhebeelement und ein zweites langgestrecktes Bauchhebeelement mit jeweils einem ersten Ende und
- ein erstes langgestrecktes Zugelement und ein zweites langgestrecktes Zugelement mit jeweils einem ersten Ende,
wobei der erste Zwischenbereich und der zweite Zwischenbereich jeweils ein Umlenkelement aufweisen oder wobei der erste Endbereich und der zweite Endbereich jeweils ein Umlenkelement aufweisen, wobei das erste Bauchhebeelement im Mittelbereich des Grundkörpers im getragenen Zustand oberhalb des ersten Zugelements liegt und wobei das zweite Bauchhebeelement im Mittelbereich des Grundkörpers im getragenen Zustand oberhalb des zweiten Zugelements liegt, wobei das erste Zugelement in Richtung erster Endbereich des Grundkörpers frei beweglich verläuft und das zweite Zugelement in Richtung zweiter Endbereich des Grundkörpers frei beweglich verläuft, wobei das erste Zugelement beim ersten Mittelbereich des Grundkörpers mit den zweiten Ende endet und das zweite Zugelement beim zweiten Mittelbereich des Grundkörpers mit dem zweiten Ende endet und wobei das erste Ende des ersten Zugelements und das erste Ende des zweiten Zugelements jeweils ein Umlenkelement aufweisen, wobei das erste Bauchhebeelement vom Mittelbereich des Grundkörpers zum Umlenkelement des ersten Zwischenbereichs oder des ersten Endbereichs verläuft und dort umgelenkt wird und das zweite Bauchhebeelement vom Mittelbereich des Grundkörpers zum Umlenkelement des zweiten Zwischenbereichs oder des zweiten Endbereichs verläuft und dort umgelenkt wird, wobei das erste Bauchhebeelement vom Umlenkelement des ersten Zwischenbereichs oder des ersten Endbereichs zum Umlenkelement des ersten Zugelements verläuft und dort umgelenkt wird und das zweite Bauchhebeelement vom Umlenkelement des zweiten Zwischenbereichs oder des zweiten Endbereichs zum Umlenkelement des zweiten Zugelements verläuft und dort umgelenkt wird, wobei das erste Bauchhebeelement vom Umlenkelement des ersten Zugelements in Richtung mindestens einer der Endbereiche des Grundkörpers verläuft und wobei das zweite Bauchhebeelement vom Umlenkelement des zweiten Zugelements in Richtung mindestens einer der Endbereiche des Grundkörpers verläuft, und wobei das erste Ende des ersten Bauchhebeelements und das erste Ende des zweiten Bauchhebeelements jeweils reversibel auf dem ersten Endbereich des Grundkörpers und/oder auf dem zweiten Endbereich des Grundkörpers und/oder auf dem ersten Ende des jeweils anderen Bauchhebeelements befestigbar sind.

In einer bevorzugten Ausführungsform sind das erste Bauchhebeelement und das zweite Bauchhebeelement und/oder das erste Zugelement und das zweite Zugelement jeweils mit einem zweiten Ende am Mittelbereich des Grundkörpers verbunden und wobei bevorzugt die Verbindung des zweiten Endes des ersten Bauchhebeelements mit dem Mittelbereich des Grundkörpers im getragenen Zustand oberhalb der Verbindung des zweiten Endes des ersten Zugelements mit dem Mittelbereich des Grundkörpers liegt und wobei die Verbindung des zweiten Endes des zweiten Bauchhebeelements mit dem Mittelbereich des Grundkörpers im getragenen Zustand oberhalb der Verbindung des zweiten Endes des zweiten Zugelements mit dem Mittelbereich des Grundkörpers liegt.

In einer bevorzugten Ausführungsform sind das erste und das zweite Bauchhebeelement im Mittelbereich des Grundkörpers miteinander verbunden und/oder das erste und das zweite Zugelement im Mittelbereich des Grundkörpers miteinander verbunden sind.

In einer bevorzugten Ausführungsform weisen die Bauchhebeelemente eine größere Gesamtelastizität auf als die Zugelemente.

In einer bevorzugten Ausführungsform sind die Bauchhebeelemente und/oder die Zugelemente Gurte.

In einer bevorzugten Ausführungsform sind die Umlenkelemente der Zugelemente als Schnallen ausgebildet.

In einer bevorzugten Ausführungsform weist der Grundkörper im Mittelbereich und/oder im Zwischenbereich Stabilisierungsstäbe auf.

In einer bevorzugten Ausführungsform sind die Endbereiche des Grundkörpers als Klettverschluss ausgestaltet.

In einer bevorzugten Ausführungsform sind die miteinander verbindbaren Endbereiche des Grundkörpers mindestens 15 cm lang. Die Endbereiche des Grundkörpers können auch kürzer als 15cm sein. Die Endbereiche können auch mittels eines zusätzlichen Klettstreifens variabel miteinander verbindbar sein (ähnlich einer "Fischmaulklett" Gurtverlängerung).

In einer bevorzugten Ausführungsform können dem Grundkörper Pelotten und/oder Wärmekissen und/oder Kältekissen zugeordnet sein.

Das Bauchhebeelement, kann auch unterschiedliche Bereiche mit unterschiedlicher Elastizität / Dehnung aufweisen. Das Bauchhebeelement, kann auch unterschiedliche Bereiche mit unterschiedlicher Elastizität aufweisen. Das Bauchhebeelement, kann auch unterschiedliche Bereiche mit unterschiedlicher Dehnung aufweisen. Das Bauchhebeelement, kann auch unterschiedliche Bereiche mit unterschiedlicher Elastizität und Dehnung aufweisen. Beispielsweise kann ein zweiteiliger Gurt mit unterschiedlichen Dehnungen verwendet werden.

Bevorzugt ist die erfindungsgemäße Bandage oder Orthese ein Schwangerschaftsbandage oder Schwangerschaftsorthese.

Die vorliegende Erfindung wird anhand des folgenden Beispiels und der Figuren näher erläutert, wobei dies nicht einschränkend zu verstehen ist.

Es zeigen:
Figur 1: Eine erfindungsgemäße Bandage oder Orthese, hier als Schwangerschaftsorthese, von hinten,
Figur 2: Die erfindungsgemäße Bandage oder Orthese von Figur 1 von der linken Seite,

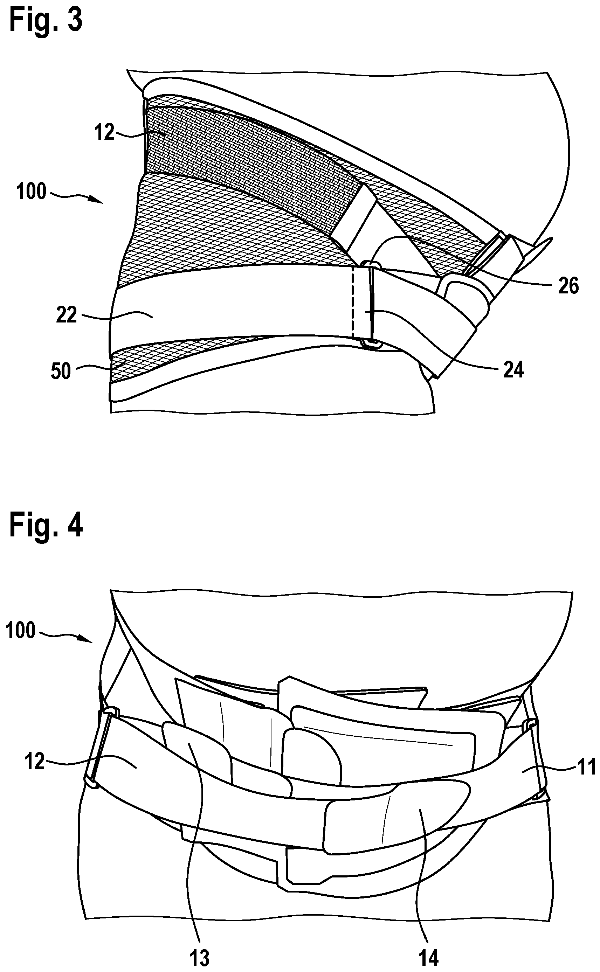

Figur 3: Die erfindungsgemäße Bandage oder Orthese von Figur 1 von der rechten Seite,
Figur 4: Die erfindungsgemäße Bandage oder Orthese von Figur 1 von vorne,
Figur 5: Die erfindungsgemäße Bandage oder Orthese von Figur 1 von der linken Seite,
Figur 6: Detailansicht einer alternativen Ausführungsform der Bandage oder Orthese,
Figur 7: Detail des Mittelbereichs einer weiteren alternativen Ausführungsform der Bandage oder Orthese von Figur 1,

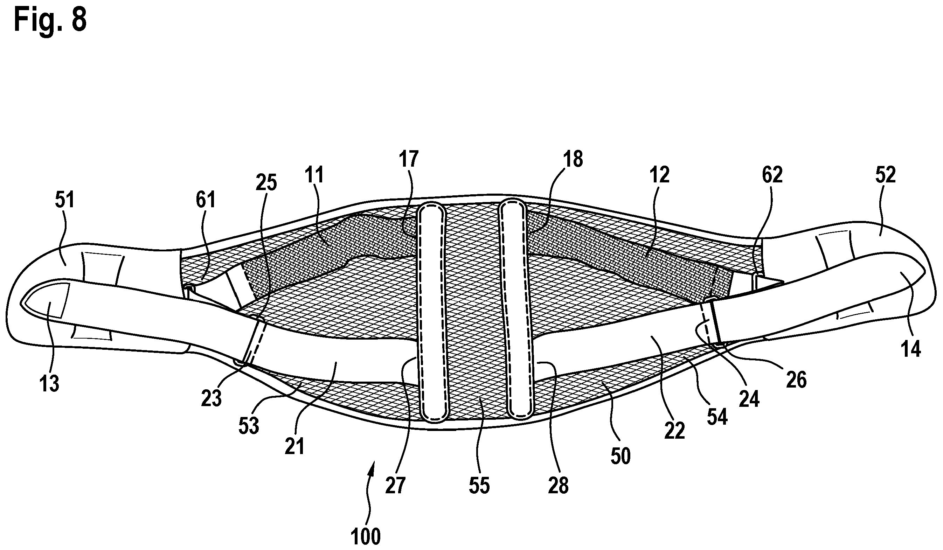

Figur 8: Gesamtansicht der Bandage oder Orthese von Figur 1.

Die Figuren zeigen eine Bandage oder Orthese (100) für den Lumbalbereich, hier in einer bevorzugten Ausführungsform einer Schwangerschaftsorthese, aufweisend einen langgestreckten, flachen Grundkörper (50), aufweisend einen ersten Endbereich (51), einen zweiten Endbereich (52), einen Mittelbereich (55) und einen ersten Zwischenbereich (53) zwischen dem ersten Endbereich (51) und dem Mittelbereich (55) und einem zweiten Zwischenbereich (54) zwischen dem zweiten Endbereich (52) und dem Mittelbereich (55), wobei der erste Endbereich (51) und der zweite Endbereich (52) miteinander reversibel verbindbar sind, ein erstes langgestrecktes Bauchhebeelement (11) und ein zweites langgestrecktes Bauchhebeelement (12) mit jeweils einem ersten Ende (13,14) und ein erstes langgestrecktes Zugelement (21) und ein zweites langgestrecktes Zugelement (22) mit jeweils einem ersten Ende (23,24), wobei der erste Zwischenbereich (53) und der zweite Zwischenbereich (54) jeweils ein Umlenkelement (61,62) aufweisen oder wobei der erste Endbereich (51) und der zweite Endbereich (52) des Grundkörpers (50) jeweils ein Umlenkelement aufweisen, wobei das erste Bauchhebeelement (11) im Mittelbereich (55) des Grundkörpers (50) im getragenen Zustand oberhalb des ersten Zugelements (21) liegt und wobei das zweite Bauchhebeelement (12) im Mittelbereich (55) des Grundkörpers (50) im getragenen Zustand oberhalb des zweiten Zugelements (22) liegt, wobei das erste Zugelement (21) in Richtung erster Endbereich (51) des Grundkörpers (50) frei beweglich verläuft und das zweite Zugelement (22) in Richtung zweiter Endbereich (52) des Grundkörpers (50) frei beweglich verläuft, wobei das erste Zugelement (21) beim ersten Zwischenbereich (53) des Grundkörpers (50) mit den ersten Ende (23) endet und das zweite Zugelement (22) beim zweiten Zwischenbereich (54) des Grundkörpers (50) mit dem ersten Ende (24) endet und wobei das erste Ende (23) des ersten Zugelements (21) und das erste Ende (24) des zweiten Zugelements (22) jeweils ein Umlenkelement (25,26) aufweisen, wobei das erste Bauchhebeelement (11) vom Mittelbereich (55) des Grundkörpers (50) zum Umlenkelement (61) des ersten Zwischenbereichs (53) oder des ersten Endbereichs (51) verläuft und dort umgelenkt wird und das zweite Bauchhebeelement (12) vom Mittelbereich (55) des Grundkörpers (50) zum Umlenkelement (62) des zweiten Zwischenbereichs (54) oder des zweiten Endbereichs (52) verläuft und dort umgelenkt wird, wobei das erste Bauchhebeelement (11) vom Umlenkelement (61) des ersten Zwischenbereichs (53) oder des ersten Endbereichs (51) zum Umlenkelement (25) des ersten Zugelements (21) verläuft und dort umgelenkt wird und das zweite Bauchhebeelement (12) vom Umlenkelement (62) des zweiten Zwischenbereichs (54) oder des zweiten Endbereichs (52) zum Umlenkelement (26) des zweiten Zugelements (22) verläuft und dort umgelenkt wird, wobei das erste Bauchhebeelement (11) vom Umlenkelement (25) des ersten Zugelements (21) in Richtung mindestens einer der Endbereiche (51,52) des Grundkörpers (50) verläuft und wobei das zweite Bauchhebeelement (12) vom Umlenkelement (26) des zweiten Zugelements (22) in Richtung mindestens einer der Endbereiche (51,52) des Grundkörpers (50) verläuft, und wobei das erste Ende (13) des ersten Bauchhebeelements (11) und das erste Ende (14) des zweiten Bauchhebeelements (12) jeweils reversibel auf dem ersten Endbereich (51) des Grundkörpers (50) und/oder auf dem zweiten Endbereich (52) des Grundkörpers (50) und/oder auf dem ersten Ende (14,13) des jeweils anderen Bauchhebeelements (12,11) befestigbar sind. Das erste Bauchhebeelement (11) und das zweite Bauchhebeelement (12) und/oder das erste Zugelement (21) und das zweite Zugelement (22) sind jeweils mit einem zweiten Ende (17, 18, 27, 28) am Mittelbereich (55) des Grundkörpers (50) verbunden wobei die Verbindung des zweiten Endes (17) des ersten Bauchhebeelements (11) mit dem Mittelbereich (55) des Grundkörpers (50) im getragenen Zustand oberhalb der Verbindung des zweiten Endes (27) des ersten Zugelements (21) mit dem Mittelbereich (55) des Grundkörpers (50) liegt und wobei die Verbindung des zweiten Endes (18) des zweiten Bauchhebeelements (12) mit dem Mittelbereich (55) des Grundkörpers (50) im getragenen Zustand oberhalb der Verbindung des zweiten Endes (28) des zweiten Zugelements (22) mit dem Mittelbereich (55) des Grundkörpers (50) liegt. Der Grundkörper (50) kann im Mittelbereich und oder im Zwischenbereich Stabilisierungsstäbe (71,72) aufweisen.

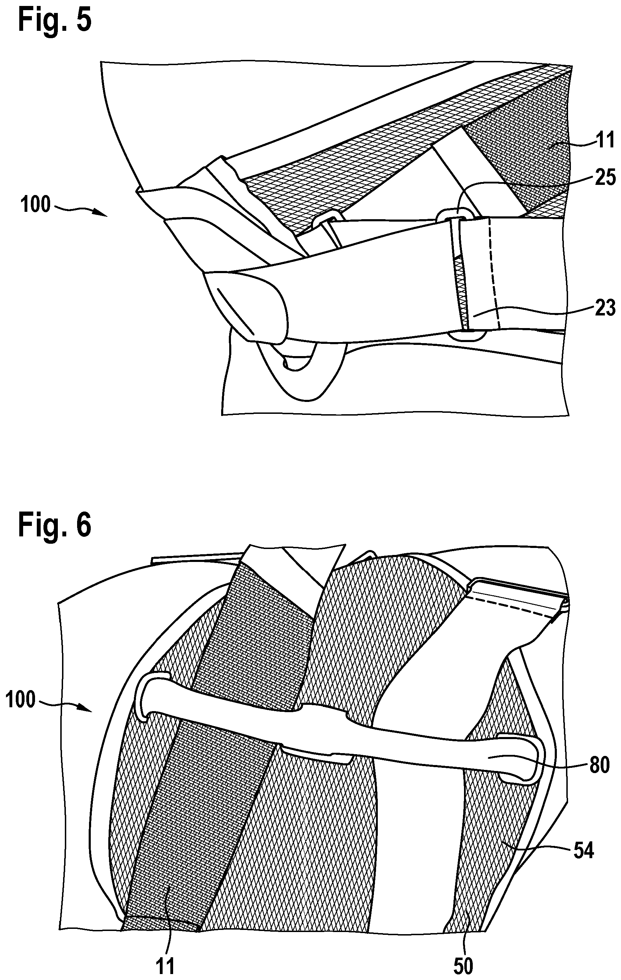

In der in Figur 6 gezeigten Ausführungsform sind das erste und das zweite Bauchhebeelement im Mittelbereich des Grundkörpers miteinander verbunden und das erste und das zweite Zugelement sind im Mittelbereich des Grundkörpers miteinander verbunden. Die Bauchhebeelemente und die Zugelemente werden durch an dem Grundkörper befindliche Führungselemente (80) geführt und gehalten.

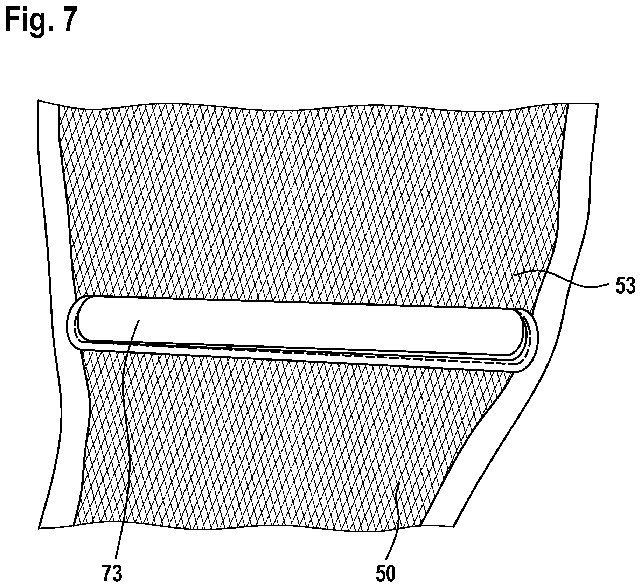

In Figur 7 ist eine weitere fakultative Ausführungsform zu sehen, in der weiter Stabilisierungsstäbe (73) vorgesehen sind und zwar in den Zwischenbereichen (53). Natürlich befindet sich dabei ein ebensolcher zusätzlicher Stabilisierungsstab auch im zweiten Zwischenbereich.

Um die Wirbelsäule und das Iliosakralgelenk sowie die Symphyse zu entlasten kann der Bauch mittels der erfindungsgemäßen Schwangerschaftsorthese "angehoben" und leicht zur Wirbelsäule zurückgeführt werden, um das entstandene übermäßige Hohlkreuz etwas auszugleichen - insbesondere vom Unterbauch her gestützt werden. Diese Abstützung leistet der neue elastische, obere der beiden Gurte, der quasi das Widerlager für den gegenüberliegenden Unterbauch bildet. Zusätzlich soll das Becken stabilisiert werden, um die ISG-Gelenk- und Symphysenschmerzen zu lindern. Diese Aufgabe kommt dem zweiten, unteren und unelastischen Gurt zu. Die Orthese erfüllt somit zwei Aufgaben getrennt voneinander. Durch das erfindungsgemäße Prinzip der Kombination elastischer und unelastischer Zuggurte bzw. Gurtanteile, deren gleitender Befestigung aufeinander und der 180-Grad Richtungsumkehr bei der Krafteinleitung gelingt es erstmals, die eingetragene Spannung primär am Unterbauch wirken zu lassen. Die drei Angriffspunkte unserer neuen Rückenorthese sind der Obere- und Untere Gurtansatz am Rücken, sowie die Bauchpartie der Orthese.

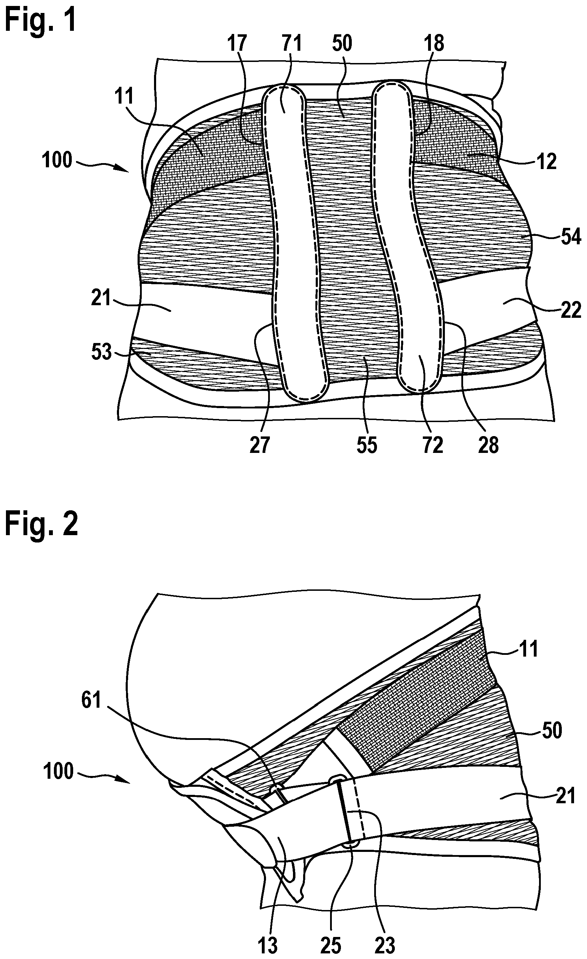

Aufbau der beispielhaften Schwangerschaftsorthese:
A. Grundkörper: Umlaufender, löchriger Grundgurt aus elastischem Gewirk. Der Grundgurt besteht aus einem flexiblem Trägermaterial. Im Bereich der Wirbelsäule sind bevorzugt parallel zueinander zwei anatomisch geformten Stabilisierungsstäbe eingebracht. Diese sind für die Funktion des neuen Hilfsmittels nicht zwingend, jedoch heutzutage üblich. Die Stäbe haben den Effekt, dass die Orthese in ihrer Höhe aufgespannt wird und nicht in sich zusammenfallen kann. Zusätzlich dienen Sie als "Ankerpunkte" für das Zuggurtsystem. Bitte beachten Sie, dass die Zuggurte auch ohne die Stäbe dort repositionierbar befestigt sein könnten, wo die Stäbe positioniert sind - die Funktion der neuen Orthese ist auch ohne die beiden Stäbe sichergestellt. Die Stäbe sind in Figur 1 zu sehen. Der zwischen beiden Stäben liegende elastische Grundgurtbereich könnte alternativ auch unelastisch ausgebildet werden, ohne wesentliche Funktionsbeeinträchtigungen zu zeitigen. Im vorliegenden Beispiel bringt dieser elastische Bereich eine bessere Größenabdeckung und eine bessere Anpassung an die Körperform. Anstelle der Stäbe könnten die Gurte auch über Gurtführungen auf dem Grundgurt fixiert werden. Bevorzugt ist auch, dass die beiden Zuggurte auch umlaufend ausgebildet sein könnten, also nicht im Bereich der Wirbelsäule getrennt und festgelegt sein müssten.
B. Bauchhebeelemente: Oberer Gurt (Gurt 1) Der "Obere elastische Gurt" oder Gurt 1 hat die Aufgabe den Bauch anzuheben. Er verläuft von der Wirbelsäule ausgehend in Richtung des Bauchverschlusses (vgl. Figur 2). Am Bauchverschluss wird er umgelenkt und verläuft wieder in Richtung der Wirbelsäule. Hierbei verläuft der Gurt jedoch in einem anderen Winkel als seinem Eingangswinkel, woraus sich eine V-Form des Gurtverlaufs im Umlenkbereich ergibt. Auf seinem Weg in Richtung der Wirbelsäule trifft der Obere Gurt auf den Unteren Gurt, an dessen Ende ein weiteres Umlenkelement befestigt ist. In diesem zweiten Umlenkelement wird der obere Gurt ein zweites Mal umgelenkt, was zu einem Z-förmigen Verlauf führt. Nach dieser zweiten Umlenkung läuft der obere Gurt erneut in Richtung Bauchverschluss. Das freie Ende des oberen Gurtes wird anschließend auf dem Bauchverschluss mittels Klett befestigt. Es können auch andere Arten der Befestigung hierzu verwendet werden. Der Gurt 1 muss aus flexiblerem Material bestehen als der Gurt 2. Wie in den Figuren zu sehen ist, kann der Gurt 1 ein oder mehrere elastische und unelastische Abschnitte in seinem Verlauf aufweisen. Vorzugsweise ist ein erster Bereich des Gurt 1 beginnend von der Wirbelsäule elastisch. Im Bereich der Umlenkungen ist ein weniger elastischer Gurt von Vorteil, da ein solcher besser durch die Umlenkungen gleitet. Die Gurte können auch als Seilzüge, Bowdenzüge, Flachbänder oder ähnliches umgesetzt werden. Es können zusätzliche Gurtführungen oder Stabilisierungsstäbe an der Orthese angebracht werden um das Trägermaterial besser aufzuspannen, dies ist in den Figuren 6 & 7 zu sehen. Die Elastizität des Gurtes ermöglicht ein "federn" des Bauches, beispielsweise beim Treppensteigen.
C. Zuggurte: Unterer Gurt (Gurt 2) Der "Untere Gurt" oder Zuggurt 2 mit seiner Funktion das Becken zu stabilisieren, kann bevorzugt eine geringere Elastizität als der Gurt 1 aufweisen. Der Gurt 2 verläuft von seiner Fixierung im Bereich der Wirbelsäule aus in Richtung des Bauchverschlusses. Der Gurt 2 endet jedoch bereits vor dem Bauchverschluss, indem er mittels Umlenkelement direkt auf dem Zuggurt 1 festgelegt ist. An seinem Ende befindet sich ein Umlenkelement in dem der Gurt 1 umgelenkt wird. Der Gurt 1 spannt somit den Gurt 2. Dieses "Y-förmige" Gurtspannprinzip mit Umlenkelement bringt den wesentlichen Vorteil, dass der 2. Zuggurt bei Eintrag einer Zugkraft auf dem elastischen Zuggurt verrutschen und so die ideale Position finden kann.

Anlegen der Schwangerschaftsorthese: Im ersten Schritt wird der Bauchverschluss des Grundträgers auf der Unterseite des Bauches geschlossen. Hierbei ist der Klettbereich des Bauchverschlusses so gestaltet, dass die Orthese während der Schwangerschaft praktisch "mitwachsen" kann, weil der Wirkbereich des Klettverschlusses gut 20 cm Umfang abdeckt. In einem zweiten Schritt wird der elastische Zuggurt 1 ca. im 90° Grad Winkel vom Körper weggezogen und damit gespannt. Dies sorgt für die Einstellung der Bauchhebefunktion, da in dieser Spannposition zunächst nur der Zuggurt 1 gespannt wird. Ist die gewünschte Spannung erreicht, wird von der 90° Grad-Position ausgehend der Zuggurt 1 auf dem Bauchverschluss festgelegt. Bei dieser Winkeländerung wird die eingetragen Zugkraft nun auch in den unelastischen Zuggurt 2 übertragen. Sind die Enden des Zuggurtes 1 auf dem Grundgurt festgelegt, ist das komplette System gespannt und die zirkuläre Kraft in beiden Zuggurten umlaufend "ausgeglichen". Gleichzeitig versperrt sich der elastische Zuggurt 1 durch die 180-Grad Umlenkung selbst und kann nicht weiter gespannt werden. Somit kann das Becken mit Hilfe des Zuggurtes 1 wie gewünscht stabilisiert werden. Je weiter die Gurtenden nun in Richtung Bauchverschluss gezogen werden, desto mehr wird A) der Unterbauch angehoben und B) das Becken stabilisiert.

Träger-Grundgurt (derzeit: netzartiges Gewirk), welcher elastisch oder unelastisch sein kann. In der bevorzugten Ausführungsform handelt es sich um ein elastisches Trägermaterial für die bessere Passform am Körper. Das Trägermaterial kann unterschiedliche Zonen mit sich unterscheidender Elastizität aufweisen. Eine Alternative Gestaltung sieht unelastische Zonen im Bereich der Wirbelsäule vor. Der Grundgurt kann im Rückenbereich mit und ohne vertikale Stabilisierungsstäbe versehen werden. Das ergänzende Zuggurtsystem bestehend aus zwei zirkulär umlaufenden Zuggurten (Oberer und Unterer Gurt) mit je zwei Umlenkelementen, wobei einer der Gurte ein Umlenkelement an seinen beiden Enden aufweist. Der andere Gurt verfügt über Klett oder ähnliche Befestigungsmittel an seinen Enden. Beide Gurte sind im Bereich der Wirbelsäule am Trägermaterial befestigt. Der Gurt 1 "Oberer Gurt" wird zweimal umgelenkt, woraus sich ein Z-förmiger Gurtverlauf ergibt. Die Erste Umlenkung erfolgt im Bereich des Bauchverschlusses. Die Zweite Umlenkung erfolgt über den Gurt 2 "Unterer Gurt".

Der Gurt 1 "Oberer Gurt", also das Bauchhebeelement kann Zonen mit unterschiedlicher Elastizität aufweisen. Unabhängig von der Elastizität der einzelnen Zonen ist der Gurt 1 genauso elastisch wie der Gurt 2 oder elastischer als der Gurt 2 ausgebildet. Der Gurt 1 kann auch einstückig aus elastischem Material sein.

Der Gurt 2 "Unterer Gurt", also das Zugelement ist bevorzugt unelastisch. Er ist im Bereich der Wirbelsäule und unterhalb des Gurt 1 angebracht. An seinem freien Ende ist ein Umlenkelement angebracht, durch welches der Gurt 1 verläuft.

Die Orthese verfügt über zumindest ein Gurtsystem. Für die bessere Anwendung und Symmetrie sind vorzugsweise zwei Gurtsysteme vorhanden. Somit befindet sich jeweils ein Gurtsystem auf jeder Seite der Orthese.

Die Umlenkelemente können in ihren Gleitflächen leicht "verzahnt" sein, um ein "rutschen" des Gurtes unter bestimmten Zugwinkeln zu erschweren. Somit wird das selektive Spannen der Gurte 1 & 2 verstärkt.

Der unelastische untere Zuggurt 2 ist über eine Schnalle mit dem elastischen Zuggurt 1 verbunden. Diese ein "Mitlaufen" des unelastischen Zuggurts 2 ermöglichende Verbindung ist das wesentliche Element der erfindungsgemäßen Schwangerschaftsorthese.

Es können Stabilisierungsstäbe an verschiedenen Positionen der Orthese angebracht werden. Zum einen mit der Funktion das Orthesen-Trägermaterial aufzuspannen. Zum anderen können somit Kräfte der Gurte besser auf die Orthese übertragen werden.

An den Enden des Trägermaterials sind Bauchverschluss-Elemente befestigt. Diese ermöglichen es, die Orthese zu schließen und bieten etwas Spielraum für den wachsenden Bauchumfang.

Die Einbindung von Pelotten ist als Aufrüstoption möglich. Es können zusätzlich oder alternativ Kälte- oder Wärmekissen in die Bandage oder Orthese integriert werden.

## Patentansprüche

1. Orthese oder Bandage (100) für den Lumbalbereich, aufweisend
- einen langgestreckten, flachen Grundkörper (50), aufweisend einen ersten Endbereich (51), einen zweiten Endbereich (52), einen Mittelbereich (55) und einen ersten Zwischenbereich (53) zwischen dem ersten Endbereich (51) und dem Mittelbereich (55) und einem zweiten Zwischenbereich (54) zwischen dem zweiten Endbereich (52) und dem Mittelbereich (55), wobei der erste Endbereich (51) und der zweite Endbereich (52) miteinander reversibel verbindbar sind,
- ein erstes langgestrecktes Bauchhebeelement (11) und ein zweites langgestrecktes Bauchhebeelement (12) mit jeweils einem ersten Ende (13,14) und
- ein erstes langgestrecktes Zugelement (21) und ein zweites langgestrecktes Zugelement (22) mit jeweils einem ersten Ende (23,24), **dadurch gekennzeichnet,**
**dass** der erste Zwischenbereich (53) und der zweite Zwischenbereich (54) jeweils ein Umlenkelement (61,62) aufweisen oder wobei der erste Endbereich (51) und der zweite Endbereich (52) des Grundkörpers (50) jeweils ein Umlenkelement aufweisen,
wobei das erste Bauchhebeelement (11) im Mittelbereich (55) des Grundkörpers (50) im getragenen Zustand oberhalb des ersten Zugelements (21) liegt und wobei das zweite Bauchhebeelement (12) im Mittelbereich (55) des Grundkörpers (50) im getragenen Zustand oberhalb des zweiten Zugelements (22) liegt,
wobei das erste Zugelement (21) in Richtung erster Endbereich (51) des Grundkörpers (50) frei beweglich verläuft und das zweite Zugelement (22) in Richtung zweiter Endbereich (52) des Grundkörpers (50) frei beweglich verläuft, wobei das erste Zugelement (21) beim ersten Zwischenbereich (53) des Grundkörpers (50) mit dem ersten Ende (23) endet und das zweite Zugelement (22) beim zweiten Zwischenbereich (54) des Grundkörpers (50) mit dem ersten Ende (24) endet und wobei das erste Ende (23) des ersten Zugelements (21) und das erste Ende (24) des zweiten Zugelements (22) jeweils ein Umlenkelement (25,26) aufweisen,
wobei das erste Bauchhebeelement (11) vom Mittelbereich (55) des Grundkörpers (50) zum Umlenkelement (61) des ersten Zwischenbereichs (53) oder des ersten Endbereichs (51) verläuft und dort umgelenkt wird und das zweite Bauchhebeelement (12) vom Mittelbereich (55) des Grundkörpers (50) zum Umlenkelement (62) des zweiten Zwischenbereichs (54) oder des zweiten Endbereichs (52) verläuft und dort umgelenkt wird,
wobei das erste Bauchhebeelement (11) vom Umlenkelement (61) des ersten Zwischenbereichs (53) oder des ersten Endbereichs (51) zum Umlenkelement (25) des ersten Zugelements (21) verläuft und dort umgelenkt wird und das zweite Bauchhebeelement (12) vom Umlenkelement (62) des zweiten Zwischenbereichs (54) oder des zweiten Endbereichs (52) zum Umlenkelement (26) des zweiten Zugelements (22) verläuft und dort umgelenkt wird,
wobei das erste Bauchhebeelement (11) vom Umlenkelement (25) des ersten Zugelements (21) in Richtung mindestens einer der Endbereiche (51,52) des Grundkörpers (50) verläuft und wobei das zweite Bauchhebeelement (12) vom Umlenkelement (26) des zweiten Zugelements (22) in Richtung mindestens einer der Endbereiche (51,52) des Grundkörpers (50) verläuft,
und wobei das erste Ende (13) des ersten Bauchhebeelements (11) und das erste Ende (14) des zweiten Bauchhebeelements (12) jeweils reversibel auf dem ersten Endbereich (51) des Grundkörpers (50) und/oder auf dem zweiten Endbereich (52) des Grundkörpers (50) und/oder auf dem ersten Ende (14,13) des jeweils anderen Bauchhebeelements (12,11) befestigbar sind.

2. Orthese oder Bandage nach Anspruch 1, wobei das erste Bauchhebeelement (11) und das zweite Bauchhebeelement (12) und/oder das erste Zugelement (21) und das zweite Zugelement (22) jeweils mit einem zweiten Ende (17, 18, 27, 28) am Mittelbereich (55) des Grundkörpers (50) verbunden sind und wobei bevorzugt die Verbindung des zweiten Endes (17) des ersten Bauchhebeelements (11) mit dem Mittelbereich (55) des Grundkörpers (50) im getragenen Zustand oberhalb der Verbindung des zweiten Endes (27) des ersten Zugelements (21) mit dem Mittelbereich (55) des Grundkörpers (50) liegt und wobei die Verbindung des zweiten Endes (18) des zweiten Bauchhebeelements (12) mit dem Mittelbereich (55) des Grundkörpers (50) im getragenen Zustand oberhalb der Verbindung des zweiten Endes (28) des zweiten Zugelements (22) mit dem Mittelbereich (55) des Grundkörpers (50) liegt.

3. Orthese oder Bandage nach einem der vorhergehenden Ansprüche, wobei das erste und das zweite Bauchhebeelement (11,12) im Mittelbereich des Grundkörpers (50) miteinander verbunden sind und/oder das erste und das zweite Zugelement (21,22) im Mittelbereich des Grundkörpers (50) miteinander verbunden sind.

4. Orthese oder Bandage nach einem der vorhergehenden Ansprüche, wobei die Bauchhebeelemente (11,12) eine größere Gesamtelastizität aufweisen als die Zugelemente (21,22).

5. Orthese oder Bandage nach einem der vorhergehenden Ansprüche, wobei die Bauchhebeelemente (11,12) und/oder die Zugelemente (21,22) Gurte sind.

6. Orthese oder Bandage nach einem der vorhergehenden Ansprüche, wobei die Umlenkelemente der Zugelemente (21,22) als Schnallen ausgebildet sind.

7. Orthese oder Bandage nach einem der vorhergehenden Ansprüche, wobei der Grundkörper im Mittelbereich und/oder im Zwischenbereich Stabilisierungsstäbe (71,72) aufweist.

8. Orthese oder Bandage nach einem der vorhergehenden Ansprüche, wobei die Endbereiche des Grundkörpers (50) als Klettverschluss ausgestaltet sind.

9. Orthese oder Bandage nach einem der vorhergehenden Ansprüche, wobei dem Grundkörper (50) Pelotten und/oder Wärmekissen und/oder Kältekissen zugeordnet sein können.

10. Orthese oder Bandage nach einem der vorhergehenden Ansprüche, wobei mindestens ein Bauchhebeelement, insbesondere jeweils beide Bauchhebeelemente (11,12), unterschiedliche Bereiche mit unterschiedlicher Elastizität und/oder Dehnung aufweist.

11. Orthese oder Bandage nach einem der vorhergehenden Ansprüche, wobei die Orthese oder Bandage eine Schwangerschaftsorthese oder eine Schwangerschaftsbandage ist.

## Claims

1. Orthosis or bandage (100) for the lumbar region, comprising
- an elongated, flat base body (50), comprising a first end region (51), a second end region (52), a central region (55) and a first intermediate region (53) between the first end region (51) and the central region (55) and a second intermediate region (54) between the second end region (52) and the central region (55), wherein the first end region (51) and the second end region (52) are reversibly connectable to each other,
- a first elongated abdominal lifting element (11) and a second elongated abdominal lifting element (12), each with a first end (13,14), and
- a first elongated tensioning element (21) and a second elongated tensioning element (22), each with a first end (23,24), **characterised in**
**that** the first intermediate region (53) and the second intermediate region (54) each comprise a deflecting element (61,62) or wherein the first end region (51) and the second end region (52) of the base body (50) each comprise a deflecting element,
wherein the first abdominal lifting element (11) is located in the central region (55) of the base body (50) in the worn state above the first tensioning element (21) and wherein the second abdominal lifting element (12) is located in the central region (55) of the base body (50) in the worn state above the second tensioning element (22),
wherein the first tensioning element (21) runs freely movable in the direction of the first end region (51) of the base body (50) and the second tensioning element (22) runs freely movable in the direction of the second end region (52) of the base body (50), wherein the first tensioning element (21) ends with the first end (23) at the first intermediate region (53) of the base body (50), and the second tensioning element (22) ends with the first end (24) at the second intermediate region (54) of the base body (50), and wherein the first end (23) of the first tensioning element (21) and the first end (24) of the second tensioning element (22) each comprise a deflecting element (25,26),
wherein the first abdominal lifting element (11) runs from the central region (55) of the base body (50) to the deflecting element (61) of the first intermediate region (53) or of the first end region (51) and is deflected there, and the second abdominal lifting element (12) runs from the central region (55) of the base body (50) to the deflecting element (62) of the second intermediate region (54) or of the second end region (52) and is deflected there,
wherein the first abdominal lifting element (11) runs from the deflecting element (61) of the first intermediate region (53) or of the first end region (51) to the deflecting element (25) of the first tensioning element (21) and is deflected there, and the second abdominal lifting element (12) runs from the deflecting element (62) of the second intermediate region (54) or of the second end region (52) to the deflecting element (26) of the second tensioning element (22) and is deflected there,
wherein the first abdominal lifting element (11) runs from the deflecting element (25) of the first tensioning element (21) in the direction of at least one of the end regions (51,52) of the base body (50) and wherein the second abdominal lifting element (12) runs from the deflecting element (26) of the second tensioning element (22) in the direction of at least one of the end regions (51,52) of the base body (50),
and wherein the first end (13) of the first abdominal lifting element (11) and the first end (14) of the second abdominal lifting element (12) are each reversibly attachable onto the first end region (51) of the base body (50) and/or onto the second end region (52) of the base body (50) and/or onto the first end (14,13) of the other abdominal lifting element (12,11) respectively.

2. Orthosis or bandage of claim 1, wherein the first abdominal lifting element (11) and the second abdominal lifting element (12) and/or the first tensioning element (21) and the second tensioning element (22) are each connected with a second end (17, 18, 27, 28) to the central region (55) of the base body and wherein preferably the connection of the second end (17) of the first abdominal lifting element (11) to the central region (55) of the base body (50) in the worn state is located above the connection of the second end (27) of the first tensioning element (21) to the central region (55) of the base body (50) and wherein the connection of the second end (18) of the second abdominal lifting element (12) to the central region (55) of the base body (50) in the worn state is located above the connection of the second end (28) of the second tensioning element (22) to the central region (55) of the base body (50).

3. Orthosis or bandage of one of the preceding claims, wherein the first and the second abdominal lifting elements (11,12) are connected to each other in the central region of the base body (50) and/or the first and the second tensioning elements (21,22) are connected to each other in the central region of the base body (50).

4. Orthosis or bandage of one of the preceding claims, wherein the abdominal lifting elements (11,12) comprise a greater total elasticity than the tensioning elements (21,22).

5. Orthosis or bandage of one of the preceding claims, wherein the abdominal lifting elements (11,12) and/or the tensioning elements (21,22) are straps.

6. Orthosis or bandage of one of the preceding claims, wherein the deflecting elements of the tensioning elements (21,22) are configured as buckles.

7. Orthosis or bandage of one of the preceding claims, wherein the base body comprises stabilising rods (71,72) in the central region and/or in the intermediate region.

8. Orthosis or bandage of one of the preceding claims, wherein the end regions of the base body (50) are designed as a hook-and-loop fastener.

9. Orthosis or bandage of one of the preceding claims, wherein pads and/or heat cushions and/or cold cushions can be assigned to the base body (50).

10. Orthosis or bandage of one of the preceding claims, wherein at least one abdominal lifting element, in particular each of the two abdominal lifting elements (11,12), comprise different regions with different elasticity and/or stretch.

11. Orthosis or bandage of one of the preceding claims, wherein the orthosis or bandage is a pregnancy orthosis or a pregnancy bandage.

## Revendications

1. Orthèse ou bandeau (100) pour la région lombaire, comprenant
- un corps de base (50) plat, allongé, comprenant une première zone d'extrémité (51), une deuxième zone d'extrémité (52), une zone centrale (55) et une première zone intermédiaire (53) entre la première zone d'extrémité (51) et la zone centrale (55) et une deuxième zone intermédiaire (54) entre la deuxième zone d'extrémité (52) et la zone centrale (55), dans laquelle ou lequel la première zone d'extrémité (51) et la deuxième zone d'extrémité (52) peuvent être reliées l'une à l'autre de manière réversible,
- un premier élément de soutien abdominal (11) allongé et un deuxième élément de soutien abdominal (12) allongé avec respectivement une première extrémité (13, 14) et
- un premier élément de traction (21) allongé et un deuxième élément de traction (22) allongé avec respectivement une première extrémité (23, 24), caractérisé(e)
en ce que la première zone intermédiaire (53) et la deuxième zone intermédiaire (54) comprennent respectivement un élément de renvoi (61, 62) ou dans laquelle ou lequel la première zone d'extrémité (51) et la deuxième zone d'extrémité (52) du corps de base (50) comprennent respectivement un élément de renvoi,
dans laquelle ou lequel le premier élément de soutien abdominal (11) se situe dans la zone centrale (55) du corps de base (50) à l'état porté au-dessus du premier élément de traction (21) et dans laquelle ou lequel le deuxième élément de soutien abdominal (12) se situe dans la zone centrale (55) du corps de base (50) à l'état porté au-dessus du deuxième élément de traction (22),
dans laquelle ou lequel le premier élément de traction (21) s'étend librement mobile dans la direction de la première zone d'extrémité (51) du corps de base (50) et le deuxième élément de traction (22) s'étend librement mobile dans la direction de la deuxième zone d'extrémité (52) du corps de base (50), dans laquelle ou lequel le premier élément de traction (21) se termine à la première zone intermédiaire (53) du corps de base (50) avec la première extrémité (23) et le deuxième élément de traction (22) se termine à la deuxième zone intermédiaire (54) du corps de base (50) avec la première extrémité (24) et dans laquelle ou lequel la première extrémité (23) du premier élément de traction (21) et la première extrémité (24) du deuxième élément de traction (22) comprennent respectivement un élément de renvoi (25, 26),
dans laquelle ou lequel le premier élément de soutien abdominal (11) s'étend de la zone centrale (55) du corps de base (50) à l'élément de renvoi (61) de la première zone intermédiaire (53) ou de la première zone d'extrémité (51) et y est renvoyé et le deuxième élément de soutien abdominal (12) s'étend de la zone centrale (55) du corps de base (50) à l'élément de renvoi (62) de la deuxième zone intermédiaire (54) ou de la deuxième zone d'extrémité (52) et y est renvoyé,
dans laquelle ou lequel le premier élément de soutien abdominal (11) s'étend de l'élément de renvoi (61) de la première zone intermédiaire (53) ou de la première zone d'extrémité (51) à l'élément de renvoi (25) du premier élément de traction (21) et y est renvoyé et le deuxième élément de soutien abdominal (12) s'étend de l'élément de renvoi (62) de la deuxième zone intermédiaire (54) ou de la deuxième zone d'extrémité (52) à l'élément de renvoi (26) du deuxième élément de traction (22) et y est renvoyé,
dans laquelle ou lequel le premier élément de soutien abdominal (11) s'étend de l'élément de renvoi (25) du premier élément de traction (21) dans la direction d'au moins l'une des zones d'extrémité (51, 52) du corps de base (50) et dans laquelle ou lequel le deuxième élément de soutien abdominal (12) s'étend de l'élément de renvoi (26) du deuxième élément de traction (22) dans la direction d'au moins l'une des zones d'extrémité (51, 52) du corps de base (50),
et dans laquelle ou lequel la première extrémité (13) du premier élément de soutien abdominal (11) et la première extrémité (14) du deuxième élément de soutien abdominal (12) peuvent être fixées respectivement de manière réversible sur la première zone d'extrémité (51) du corps de base (50) et/ou sur la deuxième zone d'extrémité (52) du corps de base (50) et/ou sur la première extrémité (14, 13) de l'autre élément de soutien abdominal (12, 11), respectivement.

2. Orthèse ou bandeau selon la revendication 1, dans laquelle ou lequel le premier élément de soutien abdominal (11) et le deuxième élément de soutien abdominal (12) et/ou le premier élément de traction (21) et le deuxième élément de traction (22) sont reliés respectivement à une deuxième extrémité (17, 18, 27, 28) au niveau de la zone centrale (55) du corps de base (50) et dans laquelle ou lequel, de préférence, la liaison de la deuxième extrémité (17) du premier élément de soutien abdominal (11) à la zone centrale (55) du corps de base (50) se situe à l'état porté au-dessus de la liaison de la deuxième extrémité (27) du premier élément de traction (21) à la zone centrale (55) du corps de base (50) et dans laquelle ou lequel la liaison de la deuxième extrémité (18) du deuxième élément de soutien abdominal (12) à la zone centrale (55) du corps de base (50) se situe à l'état porté au-dessus de la liaison de la deuxième extrémité (28) du deuxième élément de traction (22) à la zone centrale (55) du corps de base (50).

3. Orthèse ou bandeau selon l'une des revendications précédentes, dans laquelle ou lequel le premier et le deuxième élément de soutien abdominal (11, 12) sont reliés l'un à l'autre dans la zone centrale du corps de base (50) et/ou le premier et le deuxième élément de traction (21, 22) sont reliés l'un à l'autre dans la zone centrale du corps de base (50).

4. Orthèse ou bandeau selon l'une des revendications précédentes, dans laquelle ou lequel les éléments de soutien abdominal (11, 12) présentent une élasticité totale supérieure à celle des éléments de traction (21, 22).

5. Orthèse ou bandeau selon l'une des revendications précédentes, dans laquelle ou lequel les éléments de soutien abdominal (11, 12) et/ou les éléments de traction (21, 22) sont des sangles.

6. Orthèse ou bandeau selon l'une des revendications précédentes, dans laquelle ou lequel les éléments de renvoi des éléments de traction (21, 22) sont conçus sous forme de boucles.

7. Orthèse ou bandeau selon l'une des revendications précédentes, dans laquelle ou lequel le corps de base comprend dans la zone centrale et/ou dans la zone intermédiaire des tiges stabilisatrices (71, 72).

8. Orthèse ou bandeau selon l'une des revendications précédentes, dans laquelle ou lequel les zones d'extrémité du corps de base (50) sont configurées sous forme de fermeture auto-agrippante.

9. Orthèse ou bandeau selon l'une des revendications précédentes, dans laquelle ou lequel des coussinets et/ou des coussins chauffants et/ou des coussins réfrigérants peuvent être associés au corps de base (50).

10. Orthèse ou bandeau selon l'une des revendications précédentes, dans laquelle ou lequel au moins un élément de soutien abdominal, en particulier respectivement les deux éléments de soutien abdominal (11, 12), comprend différentes zones avec des élasticités différentes et/ou des allongements différents.

11. Orthèse ou bandeau selon l'une des revendications précédentes, l'orthèse ou le bandeau étant une orthèse de grossesse ou un bandeau de grossesse.
